# EUROPEAN PATENT APPLICATION

(11) **EP 2 248 794 A1**
(43) Date of publication of application: **10.11.2010**
(21) Application number: 09717938.6
(22) Date of filing: 02.03.2009
(51) Int. Cl.: C07C 29/42, C07C 17/16, C07C 21/215, C07C 33/048

(54) **PROCESS FOR PRODUCTION OF DIALCOHOL, PROCESS FOR PRODUCTION OF ALLYLHALIDE COMPOUND, AND ALLYLCHLORIDE COMPOUND**

(30) Priority: 03.03.2008 JP 2008051814
(71) Applicant: Sumitomo Chemical Company, Limited, Tokyo 104-8260 (JP)
(72) Inventor: TAKAHASHI, Toshiya, Toyonaka-shi Osaka 560-0021 (JP)
(74) Representative: Henkel, Feiler & Hänzel
(86) International application number: PCT/JP2009/053819
(87) International publication number: WO 2009/110406

(57) **Abstract**

Disclosed are: an advantageous production process for a carotenoid intermediate; and others. Specifically disclosed are: a process for producing a dialcohol represented by formula (1), which is **characterized by** reacting a Grignard reagent with an acetylene gas in an organic solvent at a temperature of 30°C or higher to prepare an ethynyl magnesium halide and subsequently reacting the ethynyl magnesium halide with methacrolein; a process for producing an allylhalide compound represented by formula (3) [wherein X represents a halogen atom: and the wavy line means the compound is either of E/Z geometric isomers or a mixture thereof], which is **characterized by** reducing a dialcohol represented by formula (1) with hydrogen to produce a triene alcohol represented by formula (2) [wherein the wavy line is as defined above] and halogenating the triene alcohol; and an allylchloride compound represented by formula (4) [wherein the wavy line is as defined above].

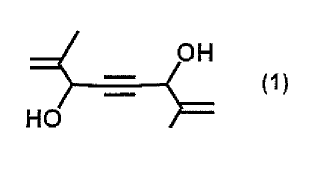

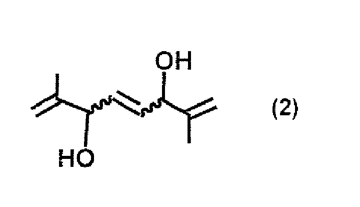

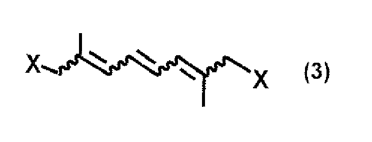

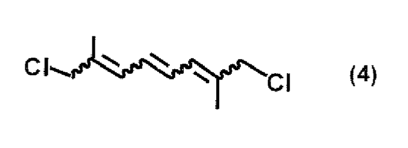

## Description

### Technical Field

The present invention relates to advantageous processes for producing intermediate compounds for producing carotenoids. More specifically, it relates to a process for producing a dialcohol, a process for producing an allylhalide compound and a process for producing an allylchloride compound.

### Background Art

A dialcohol represented by the formula (1): 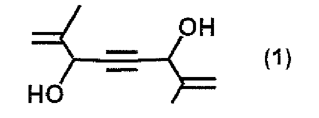 has been known as an important intermediate compound for producing one of carotinoides, β-carotene. As a synthetic process of the dialcohol, non-Patent Document 1 discloses a reaction of a Grignard reagent with an acetylene gas in diethyl ether.
non-Patent Document 1: Journal of Organic Chemistry (1961), 26, 1171-3

### Disclosure of the Invention

### Problem to be solved by the invention

However, the above synthetic process is not always easily carried out industrially. A main object of the present invention is to provide a process for simply and easily producing a dialcohol represented by the formula (1).

### Means for solving the problem

The present inventor has studied intensively to achieve the above object and has completed the present invention.
That is, the present invention is:
(1) A process for producing a dialcohol represented by the formula (1): 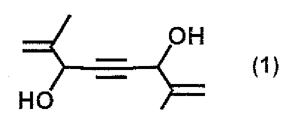 which comprises:
   the first step, wherein a Grignard reagent is reacted with an acetylene gas in an organic solvent at a temperature of 30°C or higher to obtain an ethynyl magnesium halide, and
   the second step, wherein the ethynyl magnesium halide obtained in the first step is reacted with methacrolein;

(2) The process for producing a dialcohol according to the above (1), wherein the Grignard reagent is an ethyl magnesium halide;
(3) The process for producing a dialcohol according to the above (1) or (2), wherein the organic solvent used in the first and second steps is at least one organic solvent selected from the group consisting of tetrahydrofuran, methyl t-butyl ether, and cyclopentyl methyl ether;

(4) A process for producing an allylhalide compound represented by the formula (3): 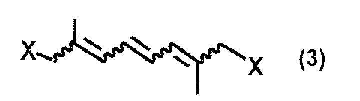 wherein X represents a halogen atom, and the wavy line represents that the compound is either of E/Z geometric isomers or a mixture thereof, which comprises:
   the third step, wherein a dialcohol represented by the formula (1): 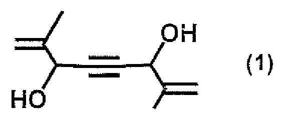 is reduced to obtain a triene alcohol of the formula (2): 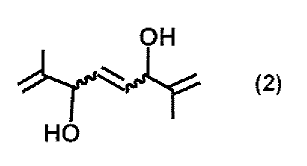 wherein the wavy line is as defined above; and
   the fourth step, wherein the triene alcohol obtained in the third step is halogenated;
(5) The process for producing an allylhalide compound according to the above (4), wherein the dialcohol represented by the formula (1) is that obtained by the process according to any one of the above (1) to (3); and
(6) An allylchloride compound represented by the formula (4): 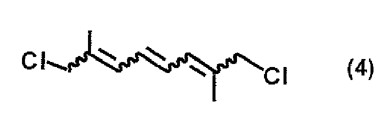 wherein the wavy line represents that the compound is either of E/Z geometric isomers or a mixture thereof.

### Effect of the invention

According to the production processes of the present invention, important intermediate compounds for producing carotenoids such as the dialcohol represented by the formula (1) can be simply and easily produced.

### Best Mode for Carrying Out the Invention

Hereinafter, the present invention will be explained in detail.
One aspect of the present invention is a process for producing a dialcohol represented by the formula (1) (hereinafter, sometimes, referred to as the dialcohol (1)) which comprises the following first and second steps.
The first step is a reaction of a Grignard reagent with an acetylene gas in an organic solvent at 30°C or higher to obtain an ethynyl magnesium halide. The second step is a reaction of the ethynyl magnesium halide obtained in the first step with methacrolein.

Examples of the Grignard reagent used in the first step include ethyl magnesium bromide, ethyl magnesium chloride, methyl magnesium bromide, methyl magnesium chloride, isopropyl magnesium bromide, and isopropyl magnesium chloride, with ethyl magnesium bromide being preferred.
The Grignard reagent is usually used in an amount of about 0.5 to 3 mol per mol of methacrolein used in the second step.

The acetylene gas used in the first step is preferably a solution-in-organic solvent type acetylene gas in an acetylene cylinder. In particular, an acetylene gas from which an organic solvent is removed by, for example, cold trap is preferred.

Methacrolein used in the second step preferably contains a polymerization inhibitor, in particular, hydroquinone. The content of the polymerization inhibitor is preferably in a range from 100 ppm to 3,000 ppm.

Examples of the organic solvent include ether solvents such as tetrahydrofuran, methyl t-butyl ether, and cyclopentyl methyl ether. The organic solvent may be a sole ether solvent or a mixed solvent of two or more ether solvents. Further, the organic solvent may be a mixed solvent of one or more ether solvents and hydrocarbon solvent(s) such as toluene and xylene.

The reaction temperature of the first step is 30°C or higher, preferably, 30 to 70°C. The temperature of 30°C or higher is preferred because it tends to improve the selectivity of the dialcohol (1) in the second step.
The reaction temperature of the second step can be appropriately selected according to a particular solvent used but, usually, it is in a range from -78°C to the boiling point of a particular solvent used, preferably, 30°C or higher.
The reaction time of each of the first and second steps varies depending on various conditions such as a particular solvent used and reaction temperature. Usually, the reaction time is in a range from about 10 minutes to 24 hours.

After termination of the second step, the dialcohol (1) can be produced by subjecting a reaction mixture to a conventional post-treatment, for example, operation such as extraction, washing, crystallization, various chromatography, and distillation off of low boiling point materials.
Further, sometimes, the reaction rate can be improved by treating the product thus obtained with an active charcoal before subjecting to the third step as described hereinafter.

The dialcohol (1) thus obtained can be used for the production of an allylhalide compound represented by the formula (3): 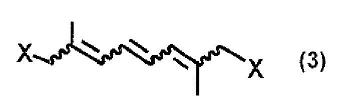 wherein X represents a halogen atom, and the wavy line represents that the compound is either of E/Z geometric isomers or a mixture thereof, in a process comprising the following third and fourth steps.

The third step is reduction of the dialcohol (1) with hydrogen to obtain a triene alcohol represented by the formula (2): 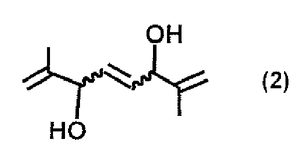 wherein the wavy line is as defined above.
The fourth step is halogenation of the triene alcohol obtained in the third step.

X in the allylhalide compound represented by the formula (3) represents a halogen atom. Specific examples thereof include a chlorine atom, a bromine atom, and an iodine atom, with a chlorine atom and a bromine atom being preferred, and a chlorine atom being more preferred. In particular, X is preferably a chlorine atom. The allylhalide compound represented by the formula (3) wherein X is a chlorine atom can be represented by the formula (4) : 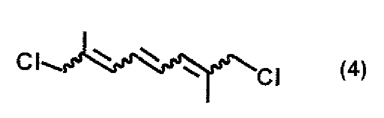 wherein the wavy line represents that the compound is either of E/Z geometric isomers or a mixture thereof.

A catalyst is used in the third step, and examples thereof include various Lindlar's catalysts.
In order to improve the reaction selectivity, for example, a base such as quinoline, or cyclohexene can be added.
The Lindlar's catalyst is usually used in an amount of 0.5 wt% to 10 wt%, and the base is used in an amount of 0.5 mol% to 10 mol% both relative to the dialcohol (1).

In order to selectively reduce the triple bond in the dialcohol (1), hydrogen in the third step is preferably supplied at low pressure of 0.5 MPa or lower, more preferably, 0.005 to 0.3 MPa. Further, preferably, the supply of hydrogen is stopped as soon as possible after absorption of the stoichiometric amount of hydrogen gas. Furthermore, it is possible to efficiently promote the reaction by supplying hydrogen gas at ordinary pressure to bubbling it into a reaction mixture.

Preferably, the third step is carried out in an organic solvent. Examples of the organic solvent used include alcohol solvents such as methanol, ethanol, isopropyl alcohol, and t-butanol; hydrocarbon solvents such as n-hexane, cyclohexane, n-pentane, benzene, toluene, and xylene; ester solvents such as ethyl acetate; aprotic nonpolar solvents such as acetonitrile, N,N-dimethylformamide, dimethylsulfoxide, hexamethylphosphoric triamide, sulfolane, 1,3-dimethyl-2-imidazolidinone, and 1-methyl-2-pyrrolidinone; and ether solvents such as diethyl ether, tetrahydrofuran, methyl t-butyl ether, cyclopentyl methyl ether, 1,4-dioxane, dimethoxyethane, anisole, diglyme, triglyme, and tetraglyme. They can be used alone or as a mixed solvent of two or more thereof.

Usually, the reaction temperature of the third step can be appropriately selected within a range from -78°C to the boiling point of a particular solvent used. However, in order to improve the selectivity of reducing reaction, the reaction temperature is 50°C or lower, preferably, 10 to 40°C.
The reaction time of the third step varies depending on various conditions such as a particular solvent used, catalyst and reaction temperature. Usually, the reaction time is in a range from about 10 minutes to 24 hours.

After termination of the third step, the triene alcohol represented by the formula (2) can be produced by subjecting a reaction mixture to a conventional post-treatment, for example, after filtrating off the catalyst, subjecting to operation such as washing, crystallization, and various chromatography. Further, after filtering off the catalyst, a reaction mixture as it is can be used for the subsequent fourth step without purification.

The halogenation in the fourth step is carried out with a halogenating agent. As the halogenating agent, for example, an aqueous solution, an alcoholic solution, or an acetic acid solution of a hydrogen halide can be used. Preferably, examples of the hydrogen halide used include HBr, HCl and HI, with HCl being particularly preferred. The halogenating agent is usually used in an amount ranging from 2 mol to 30 mol per mol of the triene alcohol represented by the formula (2).

Usually, the fourth step is carried out in an organic solvent or a mix solvent thereof with water. Examples of the organic solvent include alcohol solvents such as methanol, ethanol, isopropyl alcohol, and t-butanol; hydrocarbon solvents such as n-hexane, cyclohexane, n-pentane, benzene, toluene, and xylene; ester solvents such as ethyl acetate; aprotic nonpolar solvents such as acetonitrile, N,N-dimethylformamide, dimethylsulfoxide, hexamethylphosphoric triamide, sulfolane, 1,3-dimethyl-2-imidazolidinone, and 1-methy-1-2-pyrrolidinone; and ether solvents such as diethyl ether, tetrahydrofuran, methyl t-butyl ether, cyclopentyl methyl ether, 1,4-dioxane, dimethoxyethane, anisole, diglyme, triglyme, and tetxaglyme. They can be used alone or as a mixed solvent of two or more thereof.

Usually, the reaction temperature of the fourth step can be appropriately selected within a range from -78°C to the boiling point of a particular solvent. Preferably, the reaction is carried out at -30 to 20°C.
The reaction time of the fourth step varies depending on various conditions such as a particular solvent used, catalyst and reaction temperature. Usually, the reaction time is in a range from about 10 minutes to 24 hours.
Preferably, the fourth step is carried out in an atmosphere of an inert gas. Further, the fourth step is carried out in the presence of an antioxidant as a stabilizer such as 3,5-di-t-butyl-4-hydroxytoluene (BHT), ethoxyquin, and vitamin E.

After termination of the fourth step, the allylhalide compound represented by the formula (3) can be produced by subjecting a reaction mixture to a conventional post-treatment, for example, subjecting to operation such as, filtration, washing, crystallization, and various chromatography.

From the allylhalide compound represented by the formula (3) thus obtained, β-carotere can be derived, for example, together with a compound represented by the formula (5) under basic conditions according to the following reaction alkylation, removal reaction): 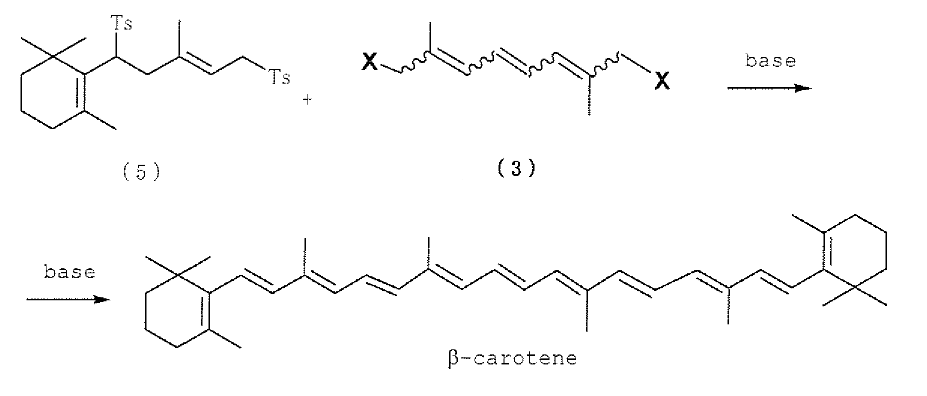 wherein Ts represents CH₂C₆H₄SO₂-. Therefore, the allylhalide compound can be regarded as an important intermediate compound for the production of carotenoids such as β-carotene.
Hereinafter, the present invention will be illustrated in more detail by means of Examples and Reference Examples, but the present invention is not limited thereto.

### Example 1

### (The first step)

A flask was purged with argon gas and 40 mL of tetrahydrofuran (hereinafter, sometimes, referred to as THF) was placed therein at 25°C. In this flask, 36.7 mL (36.7 mmol) of a separately prepared 1 mol/L ethyl magnesium bromide solution in THF was placed and the temperature was raised to 50°C. A given amount of acetylene gas was bubbled into the solution at 50 to 55°C for 3 hours and then the bubbling was ceased. Argon gas was bubbled into the reaction mixture at the same temperature for 30 minutes to expel an excess amount of acetylene gas from the system, thereby terminating the first step.

### (The second step)

In 10 mL of THF, 2.0 g (28.3 mmol) of methacrolein containing 1,000 ppm of dibutylhydroxytoluene (BHT) was dissolved. The THF solution was added dropwise to the solution obtained in the first step at 30°C over 30 minutes. The mixture was maintained at 30 to 35°C for 2 hours. After cooling to 30°C or lower, a cold saturated ammonium chloride was slowly added dropwise thereto and the mixture was extracted with ethyl acetate. The ethyl acetate layer was washed with water and saturated brine, and dehydrated over magnesium sulfate. Then, the solvent was distilled off with an evaporator to obtain a mixture of the alcohol (I) and the alcohol (II) as shown hereinafter in the ratio of 78 : 22. The yield of the alcohol (I) was 76%.

### Example 2

### (The first step)

A flask was purged with argon gas and 300 mL of THF was placed therein at 25°C. In this flask, 250 mL (250 mol) of a separately prepared 1 mol/L ethyl magnesium bromide solution in THF was placed and the temperature was raised to 50°C. A given amount of acetylene gas was bubbled into the solution at 50 to 55°C for 3 hours and then the bubbling was ceased. Argon gas was bubbled into the reaction mixture at the same temperature for 30 minutes to expel an excess amount of acetylene gas from the system, thereby terminating the first step.

### (The second step)

In 50 mL of THF, 21.45 g (300 mmol) of methacrolein containing 1,000 ppm of BHT was dissolved. The THF solution was added dropwise to the solution obtained in the first step at 30°C over 1 hour. The mixture was maintained at 30 to 35°C for 2 hours. After cooling to 30°C or lower, a cold saturated ammonium chloride was slowly added dropwise thereto and the mixture was extracted with ethyl acetate. The ethyl acetate layer was washed with water and saturated brine, and dehydrated over magnesium sulfate. Then, the solvent was distilled off with an evaporator to obtain a mixture of the alcohol (I) and the alcohol (II) in the ratio of 89 : 11. No impurity was observed by GC analysis except for these 2 components.

### Example 3

According to the same manner as that in Example 1, the reaction and post-treatment were carried out except for using cyclopentyl methyl ether instead of THF, and bubbling acetylene gas at 36°C to obtain a mixture of the alcohol (I) and the alcohol (II) in the ratio of 98 : 2. The yield of the alcohol (I) was 90%.

### Example 4

According to the same manner as that in Example 1, the reaction and post-treatment were carried out except for using methyl t-butyl ether instead of THF to obtain a mixture of the alcohol (I) and the alcohol (II) in the ratio of 72 : 28. The yield of the alcohol (I) was 64%.

### Reference Example 1

According to the same manner as that in Example 1, the first step and the second step were carried out except that the reaction temperature was 20 to 25°C to obtain a mixture of the alcohol (I) and the alcohol (II) in the ratio of 18 : 82.

### Reference Example 2

According to the same manner as that in Example 1, the first step and the second step were carried out except that the reaction temperature was 0 to 5°C to obtain a mixture of the alcohol (I) and the alcohol (II) in the ratio of 6 : 94.

### Reference Example 3

### Example of the reaction with methacrolein

According to the same manner as that in Example 1, the first step and the second step were carried out except that a mixed solvent of diethyl ether and toluene was used instead of THF and the reaction temperature was 0 to 5°C to obtain a mixture of the alcohol (I) and the alcohol (II) in the ratio of 95 : 5. The yield of the alcohol (I) was 49%. An insoluble oily material was formed in the reaction mass into which acetylene gas was bubbled, which made handling of the reaction mass difficult.

### Example 5

### (The third step)

In a flask, 550 mg (3.16 mmol) of the alcohol (I) and 70 mL of isopropyl alcohol were placed to form a solution and to the solution were added 20 mg (0.16 mmol) of quinoline and 26 mg (5 wt%) of a Lindlar's catalyst. The flask was purged with hydrogen gas and the reaction was carried out at 20 to 30°C and hydrogen pressure of 0.02 MPa for 3.5 hours. After the reaction, the catalyst was filtered off and the solvent was distilled off with an evaporator to obtain the alcohol (III) as shown hereinafter at a yield of 89%.

### Example 6

### (The third step)

According to the same manner as that in Example 3, the third step was carried out except for using toluene instead of isopropyl alcohol to obtain the alcohol (III) at a yield of 83%.

### Example 7

### (The fourth step)

In a flask, 500 mg (2.44 mmol) of the alcohol (III) and 20 mL of isopropyl alcohol were placed to form a solution and the solution was cooled to -10 to 0°C. To the solution was added dropwise 2.54 g (24.4 mmol) of 35% hydrochloric acid at the same temperature over 30 minutes, and the mixture was maintain at the same temperature for 15 minutes. Then, water was added dropwise to the mixture and the deposition of crystals was confirmed. The crystals were collected by filtration in an atmosphere of nitrogen, washed with 5% sodium hydrogen carbonate and water, and then dried to obtain the allylchloride (IV) at a yield of 85%.
The allylchloride (IV)
FD-MS m/z=204
¹H-NMR δ (CDCl₃): 1.87 (6H, s), 4.07 (4H, s), 6.16-6.18 (2H, m), 6.40-6.42 (2H, m)
¹³C-NMR δ (CDCl₃): 14.9, 52.2, 129.5, 129.6, 134.5
The data of MS and NMR showed that the main component was the allylchloride (IV).
The geometric isomerism of the terminal olefin is the trans isomer according to NOE measurement.

### Example 8

### (The fourth step)

In a flask, 300 mg (1.59 mmol) of the alcohol (III) and 150 mL of isopropyl alcohol were placed to form a solution and the solution was cooled to -10 to 0°C. To the solution was added dropwise 2.68 g (15.9 mmol) of 48% hydrobromic acid at the same temperature over 30 minutes, and the mixture was maintain at the same temperature for 15 minutes. Then, the deposition of crystals was confirmed. The crystals were collected by filtration in an atmosphere of nitrogen, washed with 5% sodium hydrogen carbonate and water, and then dried to obtain the allylbromide(V) at a yield of 80%.
The allylbromide (V)
FD-MS m/z=294
¹H-NMR δ (CDCl₃): 1.91 (6H, s), 4.06 (4H, s), 6.25-6.26 (2H, s), 6.40-6.42 (2H, m)
¹³C-NMR δ (CDCl₃): 15.4, 41.5, 130.0, 130.2, 135.0
The NMR data showed that the main component was the allylbromide (V).
The geometric isomerism of the terminal olefin is the trans isomer according to NOE measurement.

The chemical structures of respective compounds in Examples and Reference Examples are as follows.

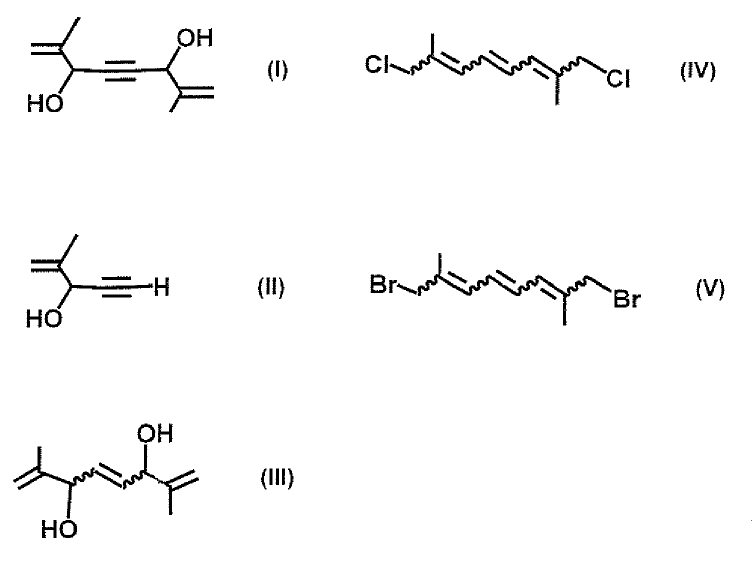

### Industrial Applicability

According to the production processes of the present invention, important intermediate compounds for producing carotenoids such as the dialcohol represented by the formula (1) can be simply and easily produced.

## Claims

1. A process for producing a dialcohol represented by the formula (1): 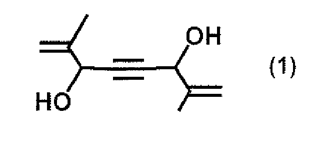 which comprises:
the first step, wherein a Grignard reagent is reacted with an acetylene gas in an organic solvent at a temperature of 30°C or higher to obtain an ethynyl magnesium halide, and
the second step, wherein the ethynyl magnesium halide obtained in the first step is reacted with methacrolein.

2. The process for producing a dialcohol according to claim 1, wherein the Grignard reagent is an ethyl magnesium halide.

3. The process for producing a dialcohol according to claim 1, wherein the organic solvent used in the first and second steps is at least one organic solvent selected from the group consisting of tetrahydrofuran, methyl t-butyl ether and cyclopentyl methyl ether.

4. A process for producing an allylhalide compound represented by the formula (3): 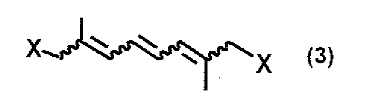 wherein X represents a halogen atom, and the wavy line represents that the compound is either of E/Z geometric isomers or a mixture thereof, which comprises:
the third step, wherein a dialcohol represented by the formula (1): 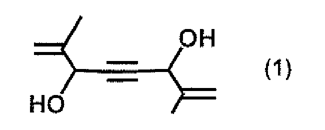 is reduced to obtain a triene alcohol of the formula (2): 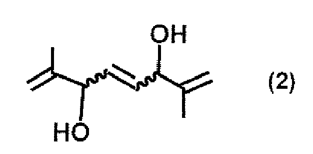 wherein the wavy line is as defined above; and
the fourth step, wherein the triene alcohol obtained in the third step is halogenated.

5. The process for producing an allylhalide compound according to claim 4, wherein the dialcohol represented by the formula (1) is that obtained by the process according to claim 1.

6. An allylchloride compound represented by the formula (4): 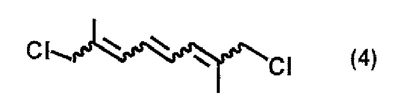 wherein the wavy line represents that the compound is either of E/Z geometric isomers or a mixture thereof.
